# NEUE EUROPÄISCHE PATENTSCHRIFT

(11) **EP 2 366 427 B2**
(45) Veröffentlichungstag und Bekanntmachung des Hinweises auf die Entscheidung über den Einspruch: **15.05.2019**
(45) Hinweis auf die Patenterteilung: 25.01.2012
(21) Anmeldenummer: 10002694.7
(22) Anmeldetag: 15.03.2010
(51) Int. Cl.: A61N 5/06, H01J 61/82

(54) **Bestrahlungsvorrichtung zur Bestrahlung der menschlichen Haut**
Irradiation device for irradiating human skin
Dispositif d'irradiation destiné à irradier la peau humaine

(43) Veröffentlichungstag der Anmeldung: 21.09.2011
(73) Patentinhaber: JK-Holding GmbH, 53578 Windhagen (DE)
(72) Erfinder: Müller, Wolfgang, 50968 Köln-Marienburg (DE)
(74) Vertreter: Sparing, Rolf Klaus

(56) Entgegenhaltungen:
- EP-A1- 1 156 512
- WO-A1-97/20596
- WO-A1-2006/013390
- WO-A1-2006/134555
- WO-A1-2010/070277
- DE-A1-102007 015 740
- DE-U1- 7 701 154
- Dictionary der OPTO CAL GmbH, Swiss calibration services, Tabellar

## Beschreibung

Die Erfindung betrifft eine Bestrahlungsvorrichtung nach dem Oberbegriff des Anspruchs 1.

Es ist bekannt, dass bestimmte Zellen in der Haut zu verstärkter Collagen- und Elastin-Bildung sowie zur Bildung von Enzymen angeregt werden können, die maßgeblich für die Hautstrukturen verantwortlich sind. Dies wird erreicht durch den Einsatz von Geräten zur Hautpflege mittels Lichtwellen im Bereich von 550 bis 700 nm. Dieser Prozess wird durch Anregung der Blutgefäßwände in der Haut erzeugt und hat eine Sauerstoffanreicherung sowie eine bessere Entgiftung der Haut zur Folge. Die Hydration nimmt zu und die Fähigkeit der Haut, Feuchtigkeit zu behalten, verbessert sich. Zunehmende Zellaktivitäten, verbesserte natürliche Zellreparatur sowie Zellerneuerungen führen zur Erzeugung eines gesünderen Hautbildes.

Um diesen Effekt in einer Sonnenbank zu reproduzieren, ist es bekannt, Niederdrucklampen in UV-Bestrahlungsgeräten einzusetzen, wobei diese Lampen derart ausgebildet sind, dass sie ein solches Spektrum aufweisen, dass sich sehr geringe Strahlungsanteile im UV-Bereich, jedoch überwiegende Anteile im Rotlichtbereich zwischen 600 und 650 nm ergeben.

Bei Untersuchungen in Verbindung mit den bekannten Niederdrucklampen ist allerdings festgestellt worden, dass letztlich keine nennenswerten Ergebnisse erzielt werden, die den vorgenannten Hautverbesserungseffekt mit sich bringen. Des Weiteren ist festgestellt worden, dass sich bei Bestrahlungsgeräten auf Basis der Niederdrucklampentechnik dahingehend ein Problem ergibt, dass diese Lampen keine hinreichende Wärme abgeben, was den Aspekt des Wohlfühlens während der Anwendung als problematisch gestaltet.

Aus der WO 97/20596 A1 geht bereits eine therapeutische Bestrahlungseinrichtung zur Bestrahlung der menschlichen Haut hervor. Die bekannte Vorrichtung weist eine Mehrzahl von Filtern auf, die Strahlung im Wellenlängenbereich zwischen 600 und 700 nm durchlässt.

Aufgabe der vorliegenden Erfindung ist es nun, eine Bestrahlungsvorrichtung der eingangs genannten Art zur Verfügung zu stellen, mit der Hautverbesserungseffekte erzielt werden und bei der sich während der Anwendung ein Gefühl des Wohlfühlens für den Nutzer ergibt.

Die vorgenannte Aufgabe wird erfindungsgemäß durch die Merkmale des Anspruchs 1 gelöst.

Im Zusammenhang mit der vorliegenden Erfindung ist festgestellt worden, dass sich die Hautverbesserungseffekte bei Verwendung der Niederdrucklampentechnik deshalb nicht einstellen, da die bekannten Lampen zwar im Wellenlängenbereich zwischen 550 nm und 700 nm Strahlung abgeben, die Lampen aber insgesamt nur Leistungswerte von 70 bis 110 W/m² haben. Im Zusammenhang mit der vorliegenden Erfindung ist erkannt worden, dass die eingangs genannten Hautverbesserungseffekte im Wellenlängenbereich zwischen 550 nm und 700 nm letztlich dann erreicht werden, wenn die Strahlungsleistung im vorgenannten Wellenbereich größer 450 W/m² ist. Diese Strahlungsleistung bezieht sich dabei auf einen Abstand von wenigstens 30 cm oder mehr von der betreffenden Lampe. Die vorgenannte Leistung lässt sich mit einer Hochdruck- oder Höchstdruck-Gasentladungslampe ohne weiteres erreichen. Da Lampen der vorgenannten Art bei einer hohen Temperatur arbeiten, kann auch eine hinreichende Wärmeentwicklung bei der Anwendung gewährleistet werden, so dass der Aspekt des Wohlfühlens des Nutzers erfüllt ist. Da Gasentladungslampen aufgrund der in der Lampe befindlichen Gase bzw. Füllstoffe üblicherweise ein Spektrum erzeugen, das deutliche Strahlungsspitzen auch unterhalb von 550 nm aufweist, ist, um Einflüsse insbesondere von UV-Strahlung auszuschließen, bevorzugt eine entsprechende Filtereinrichtung vorgesehen.

Von besonderem Vorteil im Zusammenhang mit der vorliegenden Erfindung ist es, wenn die Bestrahlungslampe in einer Reflektoreinrichtung vorgesehen ist, der eine Kühleinrichtung zugeordnet ist. Dabei weist die Reflektoreinrichtung einen Reflektor auf, in dem die Bestrahlungslampe angeordnet ist. Darüber hinaus weist die Reflektoreinrichtung bevorzugt auch die Filtereinrichtung auf.

Der Vorteil der vorliegenden Erfindung besteht darin, dass die erfindungsgemäße Reflektoreinrichtung in Form und Größe grundsätzlich einer Reflektoreinrichtung entsprechen kann, die beim Stand der Technik bei Sonnenbänken zur Gesichtsbräunung eingesetzt wird. Die bekannte Reflektoreinrichtung zur Gesichtsbräunung kann durch eine erfindungsgemäße Reflektoreinrichtung ohne weiteres ausgetauscht werden. Dies ermöglicht es, bekannte UV-Sonnenbänke erfindungsgemäß umzurüsten. Im Zusammenhang mit der Umrüstung der Reflektoreinrichtung sollten vorzugsweise dann auch die bei den Sonnenbänken vorhandenen UV-Niederdrucklampen durch die eingangs genannten Niederdrucklampen ersetzt werden, die jedenfalls im Wellenlängenbereich des Rotlichts abstrahlen.

Um den erfindungsgemäßen Hautverbesserungseffekt so gut wie möglich nutzen zu können, sollte bevorzugt weniger als 20%, vorzugsweise weniger als 15% und insbesondere weniger als 10% der gefilterten Strahlung im Wellenlängenbereich kleiner 550 nm anfallen. Damit kann der Hauptteil der Strahlung auf den Wellenlängenbereich zwischen 550 nm und 700 nm konzentriert werden.

Die Filtereinrichtung der erfindungsgemäßen Reflektoreinrichtung ist bevorzugt derart ausgebildet, dass nach Filterung mehr als 50%, vorzugsweise mehr als 70% und insbesondere mehr als 80% der Strahlungsleistung der gefilterten Strahlung im Wellenlängenbereich größer 500 nm anfällt. Bevorzugt ist es so, dass UV-Strahlung nicht oder in nur sehr geringem Umfang (jedenfalls kleiner 5%) anfällt, Eine weitere Optimierung der Strahlungsleistung im erfindungsgemäß bevorzugten Wellenlängenbereich ergibt sich dadurch, dass die Filtereinrichtung Strahlung der Bestrahlungslampe im Wellenlängenbereich kleiner 570 nm und/oder größer 750 nm, vorzugsweise größer 700 nm filtert.

Im Zusammenhang mit der vorliegenden Erfindung ist im Übrigen festgestellt worden, dass sich besonders gute Hautverbesserungseffekte dann ergeben, wenn die Strahlungsleistung der gefilterten Strahlung einer Bestrahlungslampe (gemessen im Abstand von mehr als 30 cm von der Bestrahlungslampe, insbesondere im Abstand von etwa 40 cm von der Bestrahlungslampe) durchgängig, d. h. ohne Unterbrechung
a) mehr als 2 W/m² im Wellenlängenbereich von 600 nm bis 670 nm beträgt und/oder
b) mehr als 4 W/m² im Wellenlängenbereich von 600 nm bis 660 nm beträgt und/oder
c) mehr als 6 W/m² im Wellenlängenbereich von 600 nm bis 630 nm beträgt und/oder
d) mehr als 8 W/m² im Wellenlängenbereich von 600 nm bis 620 nm beträgt und/oder
e) mehr als 10 W/m² im Wellenlängenbereich von 600 nm bis 615 nm beträgt und /oder
f) mehr als 12 W/m² im Wellenlängenbereich von 600 nm bis 610 nm beträgt und/oder
g) mehr als 14 W/m² im Wellenlängenbereich von 600 nm bis 605 nm beträgt und/oder
h) mehr als 16 W/m² im Wellenlängenbereich von 600 nm bis 604 nm beträgt und /oder
i) mehr als 17 W/m² im Wellenlängenbereich von 600 nm bis 603 nm beträgt.

Nachfolgende Leistungsangaben beziehen sich stets auf die gefilterte Strahlungsleistung gemessen in einem Abstand von wenigstens 30 cm von der Bestrahlungslampe, insbesondere im Abstand von etwa 40 cm von der Bestrahlungslampe.

Bei einer derartigen Strahlungsleistungsverteilung im relevanten Wellenlängenbereich zwischen 590 und 700 nm liegt die Strahlungsleistung größer 500 W/m², vorzugsweise größer 700 W/m² und insbesondere größer 800 W/m². Bei einer besonders bevorzugten AusKhmngsform sind im Bereich zwischen 590 nm und 700 nm ca. 900 W/m² an Strahlungsleistung erreicht worden. Bei einer derartigen Leistung im genannten Wellenlängenbereich konnten erhebliche Hautverbesserungseffekte festgestellt werden.

Im Zusammenhang mit der vorliegenden Erfindung ist vorgesehen, dass die Filtereinrichtung wenigstens eine Strahlung im Wellenlängenbereich bis 550 nm, vorzugsweise bis 570 nm und insbesondere bis 580 nm filternde Filterscheibe aufweist. Die vorgenannten Wellenlängenbereiche schließen nicht aus, dass eine Filterung grundsätzlich auch im Bereich oberhalb von 580 nm und/oder unterhalb von 600 nm vorgesehen sein kann. Mit Filterung ist dabei eine Blockung bzw. Reflektion entsprechender Strahlung gemeint. Darüber hinaus kann auch vorgesehen sein, dass die Filterscheibe Strahlung im Wellenlängenbereich oberhalb von 710 mm, insbesondere auch oberhalb von 700 nm filtert. Hierzu kann grundsätzlich auch eine weitere Filterscheibe dienen. Darüber hinaus kann vorgesehen sein, dass die Filtereinrichtung einen schräg gestellten Filterspiegel aufweist, der eine Strahlung im Wellenlängenbereich bis 550 nm, vorzugsweise bis 570 nm und insbesondere bis 580 nm und/oder im Wellenlängenbereich oberhalb von 710 nm, insbesondere oberhalb von 700 nm transmittiert und ansonsten reflektiert.

Statt eines schräggestellten Filterspiegels kann auch eine entsprechende, senkrecht zur Hauptstrahlungsrichtung angeordnete Filterscheibe vorgesehen sein, die nur im gewünschten Wellenlängenbereich von 580 nm bis 710 nm bzw. in dazwischenliegenden Bereichsintervallen für Strahlung durchlässig ist, während Strahlung in anderen Wellenlängenbereichen blockiert wird. Es versteht sich natürlich, dass auch innerhalb der zuvor genannten Bereichsgrenzen eine weitere Auswahl möglich ist, so kann beispielsweise der Bereich zwischen 590 nm und 700 nm oder der Bereich zwischen 600 nm und 700 nm für entsprechende Strahlung durchgängig sein.

Des Weiteren kann auch der Reflektor selbst zur Filtereinrichtung gehören und durch eine entsprechende Beschichtung und/oder Materialwahl Strahlung im Wellenlängenbereich bis 550 nm, vorzugweise bis 570 nm und insbesondere bis 580 nm und/oder im Wellenlängenbereich oberhalb von 710 nm, insbesondere bis 700 nm transmittieren und ansonsten reflektieren bzw. blocken. Hierdurch wird dann der gewünschte Wellenlängenbereich bereits durch den Reflektor entsprechend gefiltert.

Letztlich kommt im Zusammenhang mit der vorliegenden Erfindung ein neuer Reflektor, der entsprechend beschichtet ist, zur Anwendung, der Licht vorzugsweise im Bereich zwischen 550 und 710 nm reflektiert. Insbesondere wird UV-Licht nicht oder nur geringfügig reflektiert. Darüber hinaus wird bevorzugt ein spezieller Strahler auf Basis entsprechender magnetischer Vorschaltung - mit maximaler Leistung im sichtbaren Licht und einem optimierten Spektrum im Bereich des roten Lichts - als Lichtquelle verwendet. Dabei ist die Kühlung über die eingangs genannte Kühleinrichtung vorzugsweise derart anzupassen, dass der Strahler innerhalb der thermischen Grenzen eine Lebensdauer von mindestens 500 h erreichen kann. Des Weiteren kommt bevorzugt wenigstens eine neue Filterscheibe zum Einsatz, die im Wellenlängenbereich bevorzugt zwischen 580 nm und 700 nm eine entsprechende Strahlung filtert.

Die bevorzugte Anwendung der vorliegenden Erfindung ist, wie eingangs bereits erwähnt, bei einer sogenannten Sonnen- bzw. Bestrahlungsbank. Hierbei handelt es sich um ein sogenanntes Tunnelgerät mit Unterbau und am Unterbau angelenktem Oberteil, wobei die Reflektoreinrichtung dann zumindest im Gesichtsbereich des Oberteils vorgesehen ist. Wenngleich es grundsätzlich möglich ist, im Unterbau und/oder im Oberteil eine Mehrzahl von erfindungsgemäßen Reflektoreinrichtungen anzuordnen - so können beispielsweise unterhalb der Liegefläche im Unterbau und/oder auch im Oberteil über die gesamte Länge eine Mehrzahl entsprechender Reflektoreinrichtungen vorgesehen sein, ist es an sich bevorzugt, lediglich eine Reflektoreinheit vorzusehen, und zwar nur im Gesichtsbereich des Oberbaus. Der übrige ober- bzw. unterseitige Bereich des Tunnels ist bevorzugt mit langgestreckten Niederdrucklampen versehen, die Strahlung jedenfalls auch im Wellenlängenbereich zwischen 550 nm und 700 nm abgeben.

Da Gasentladungslampen während des Betriebes sehr heiß werden, ist, wie eingangs bereits erwähnt, der Reflektoreinrichtung eine Kühleinrichtung zugeordnet. Um insbesondere bei Sonnen- bzw. Bestrahlungsbänken bzw. Tunnelgeräten Verbrennungen in jedem Falle zu vermeiden, ist eine strahlungsdurchlässige Schutzscheibe vorgesehen, die über einen Luftspalt von der Reflektoreinrichtung beabstandet ist. Dabei ist eine weitere Kühleinrichtung zur Durchführung eines Kühlmediums durch den Luftspalt vorgesehen. Auf diese Weise lässt sich sicherstellen, dass der Bereich der Reflektoreinrichtung durch die gekühlte Schutzscheibe nicht zu heiß wird und dass es dem Nutzer ohne weiteres möglich ist, diesen Bereich zu berühren.

Es wird ausdrücklich darauf hingewiesen, dass alle vorstehend und auch nachfolgend angegebenen Bereichsangaben alle innerhalb der Bereichsgrenzen liegenden Zwischenbereiche und auch Einzelwerte umfassen, auch wenn diese im Einzelnen nicht angegeben sind. Letztlich werden alle im Einzelnen nicht angegebenen Zwischenintervalle und Einzelwerte innerhalb von angegebenen Bereichsgrenzen als erfindungswesentlich angesehen.

Weitere Merkmale, Vorteile und Anwendungsmöglichkeiten der vorliegenden Erfindung ergeben sich aus der nachfolgenden Beschreibung von Ausführungsbeispielen anhand der Zeichnung und der Zeichnung selbst. Dabei bilden alle beschriebenen und/oder bildlich dargestellten Merkmale für sich oder in beliebiger Kombination den Gegenstand der vorliegenden Erfindung, unabhängig von ihrer Zusammenfassung in den Ansprüchen oder deren Rückbeziehung.

Es zeigt
- Fig. 1: eine perspektivische, schematische Ansicht einer ersten Ausfüh- rungsform einer erfindungsgemäßen Bestrahlungsseinrichtung,
- Fig. 2: eine perspektivische, schematische Ansicht einer zweiten Aus- führungsform einer erfindungsgemäßen Bestrahlungseinrichtung,
- Fig. 3: eine schematische Ansicht einer Reflektoreinrichtung mit zuge- ordneter Kühlung,
- Fig. 4: eine schematische Darstellung einer ersten Ausführungsform ei- ner erfindungsgemäßen Reflektoreinrichtung,
- Fig. 5: eine schematische Darstellung einer zweiten Ausführungsform einer erfindungsgemäßen Reflektoreinrichtung,
- Fig. 6: eine schematische Darstellung einer dritten Ausführungsform ei- ner erfindungsgemäßen Reflektoreinrichtung,
- Fig. 7: eine schematische Darstellung einer vierten Ausführungsform ei- ner erfindungsgemäßen Reflektoreinrichtung,
- Fig. 8: eine Darstellung der Strahlungsleistung einer erfindungsgemäßen Bestrahlungsvorrichtung und einer zum Stand der Technik zählenden Bestrahlungslampe über die Wellenlänge,
- Fig. 9: eine Tabelle der Strahlungsleistung der erfindungsgemäßen Be- strahlungsvorrichtung und der zum Stand der Technik zählenden Bestrahlungslampe im Wellenlängenbereich zwischen 500 nm bis 659 nm und
- Fig. 10: eine Tabelle der Strahlungsleistung der erfindungsgemäßen Be- strahlungsvorrichtung und der zum Stand der Technik zählenden Bestrahlungslampe im Wellenlängenbereich zwischen 660 nm bis 800 nm.

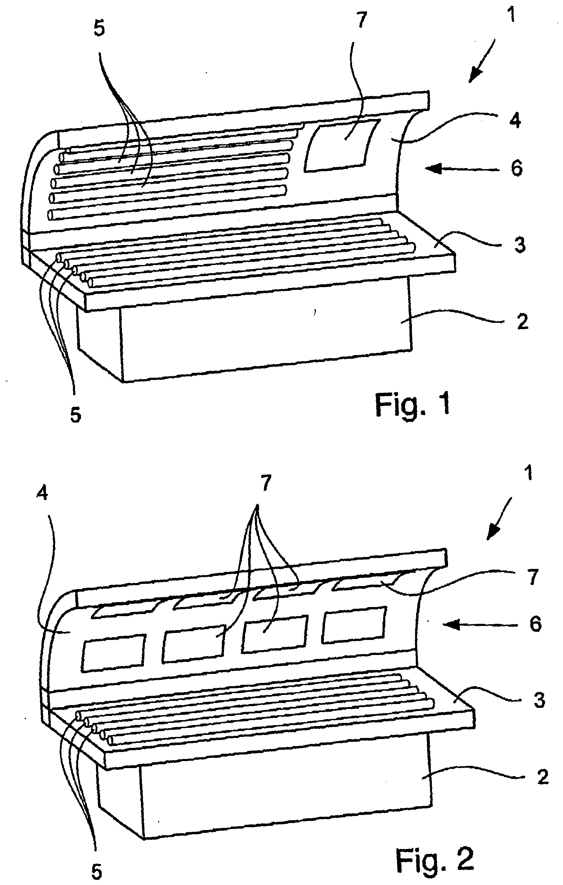

In Fig. 1 ist eine als Sonnen- oder Bestrahlungsbank ausgebildete Bestrahlungsvorrichtung 1 zur Bestrahlung der menschlichen Haut dargestellt. Bei der Bestrahlungsvorrichtung 1 handelt es sich um einen sogenanntes Tunnelgerät, das einen Unterbau 2 mit Liegefläche 3 und ein am Unterbau 2 angelenktes, verschwenkbares Oberteil 4 aufweist. Das Oberteil 4 ist auf den Unterbau 2 herabschwenkbar, so dass sich ein Tunnel ergibt, in dem sich während des Betriebes der Nutzer befindet. Unterhalb der Liegefläche 3 im Unterbau 2 und im Oberteil 4 befinden sich vorliegend langgestreckte Niederdruck-Leuchtstoff-Lampen 5, die Strahlung auch im Rotlichtbereich abgeben. Im Übrigen befinden sich im Oberteil mit Ausnahme des Gesichtsbereichs 6 ebenfalls eine Mehrzahl von Niederdruck-Leuchtstoff-Lampen 5, die auch Strahlung im Rotlichtbereich abgeben.

Im Gesichtsbereich 6 des Oberteils 4 der Bestrahlungsvorrichtung 1 befindet sich eine Reflektoreinrichtung 7. Der Reflektoreinrichtung 7 ist eine nicht dargestellte Kühleinrichtung zugeordnet. Im Übrigen weist die Reflektoreinrichtung 7 einen Reflektor 8, wenigstens eine als Hochdruck- oder Höchstdruck-Gasentladungslampe ausgebildete Bestrahlungslampe 9 und eine Filtereinrichtung 10 zur Filterung der von der Bestrahlungslampe 9 abgestrahlten Strahlung auf. Die Filtereinrichtung 10 ist vorliegend derart ausgebildet, dass Strahlung der Bestrahlungslampe 9 im Wellenlängenbereich kleiner 550 nm in jedem Falle gefiltert wird. Darüber hinaus ist die Strahlungsleistung der gefilterten Strahlung der Bestrahlungslampe 9 gemessenen im Abstand von 30 cm oder mehr von der Bestrahlungslampe 9 größer als 450 W/m².

In Fig. 2 ist eine Ausführungsform dargestellt, bei der im Bereich des Oberteils 4 der Bestrahlungsvorrichtung 1 nicht nur eine, sondern eine Mehrzahl von Reflektoreinrichtungen 7 vorgesehen sind. Im dargestellten Ausführungsbeispiel sind acht Reflektoreinrichtungen 7 vorgesehen, die paarweise angeordnet sind.

Bei einer nicht dargestellten Ausführungsform sind sowohl im Oberteil 4 als auch im Unterbau 2 jeweils eine Mehrzahl von Reflektoreinrichtungen 7 vorgesehen.

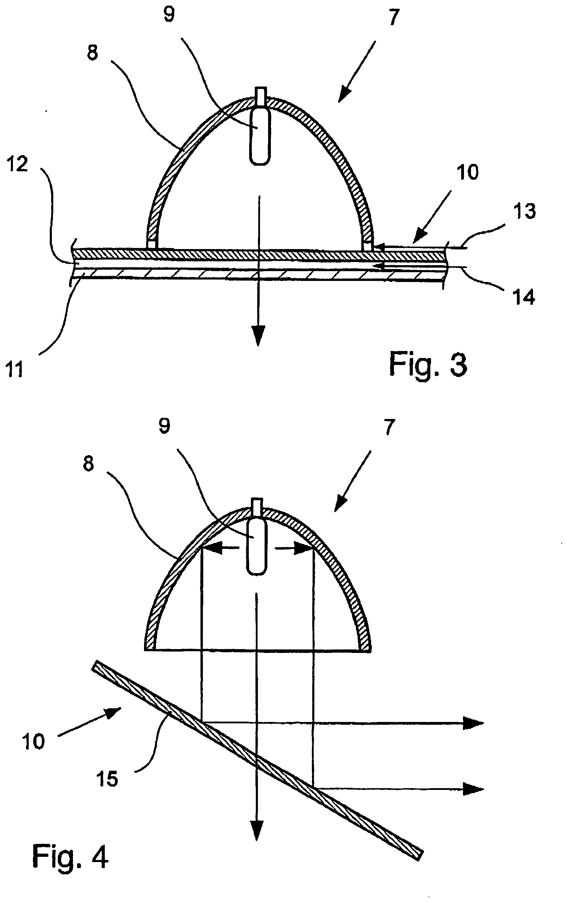

In Fig. 3 ist schematisch eine Reflektoreinrichtung 7 im in das Oberteil 4 eingebauten Zustand dargestellt. Bei der in Fig. 3 dargestellten Einbaustituation befindet sich unterhalb der Reflektoreinrichtung 7 eine strahlungsdurchlässige Schutzscheibe 11, die von der Reflektoreinrichtung 7 beabstandet ist, so dass sich ein Luftspalt 12 ergibt. Während die Reflektoreinrichtung 7 als solche über die nicht dargestellte Kühleinrichtung gekühlt wird, wird auch durch den Luftspalt 12 ein Kühlmedium geleitet, so dass die Schutzscheibe 11 keine zu hohe Temperatur erreicht. Die Kühlung als solche wird schematisch durch die Pfeile 13, 14 dargestellt. Dabei versteht es sich, dass beispielsweise die Kühlung des Reflektors nicht in der dargestellten Weise erfolgen muss. Letztlich ist durch die Kühlung 13 der Reflektoreinrichtung und die ergänzende Kühlung der Schutzscheibe 11 über den Luftspalt 12 gewährleistet, so dass die Schutzscheibe 11 eine Temperatur von 40°C nicht überschreitet.

Die Bestrahlungslampe 9 selbst ist bevorzugt als Hochdruck-Gasentladungslampe ausgebildet und weist im Betriebszustand einen Betriebsdruck von in jedem Falle mehr als 20 bar auf. Bevorzugt liegt der Betriebsdruck etwa bei 40 bar, wobei der Gasfülldruck im ausgeschalteten Zustand bei ca. 8 bar liegt. Die Zündung des in der Bestrahlungslampe 9 befindlichen Gases zur Erzeugung eines Lichtbogens erfolgt durch das Anlegen einer Zündspannung zwischen 20 und 40 kV. Die Steuerung des Zündvorgangs und des anschließendes Betriebes erfolgt über ein nicht dargestelltes konventionelles Vorschaltgerät, wobei die Zündung über ein Zündgerät erfolgt, oder über ein elektronisches Vorschaltgerät. Im Übrigen kann die Bestrahlungslampe 9 über das Vorschaltgerät auch dimmbar sein. Hierzu sind eine entsprechende Ansteuerung und Regelung sowie wenigstens ein entsprechender Schalter vorgesehen. Im Übrigen ist das Vorschaltgerät mit der Bestrahlungslampe 9 gekoppelt. Die Bestrahlungslampe 9 selbst weist ein solches Füllgas auf, das eine maximale Leistung von sichtbarem Licht mit einem optimierten Spektrum im Bereich des roten Lichts als Lichtquelle verwendet. Die Füllung der Bestrahlungslampe weist vorliegend, wie bereits erwähnt, Quecksilber auf. Des Weiteren sind kleine Anteile an Natriumjodid und Scandiumjodid vorgesehen. Die Kühlung insbesondere der Reflektoreinrichtung 7 ist derart zu wählen, dass die Betriebsdauer der Bestrahlungslampe 9 wenigstens 500 h erreicht.

Die Reflektoreinrichtung 7 weist vorliegend eine solche Filtereinrichtung 10 auf, die Strahlung der Bestrahlungslampe 9 jedenfalls im Wellenlängenbereich kleiner 550 nm filtert. Bei der in Fig. 4 dargestellten Ausführungsform weist die Reflektoreinrichtung 7 einen als Alureflektor ausgebildeten Reflektor 8 auf, dem ein schräg gestellter Filterspiegel 15, bei dem es sich bevorzugt um einen dielektrischen Spiegel handelt, zugeordnet ist. Dieser Filterspiegel 15 transmittiert Strahlung im Bereich zwischen 0 bis 580 nm und größer 710 nm, während Strahlung im Bereich zwischen 580 und 710 nm reflektiert wird. Der reflektierte Bereich kann grundsätzlich auch zwischen den Bereichsgrenzen zwischen 580 nm und 710 nm liegen, also beispielsweise zwischen 580 nm bis 700 nm oder zwischen 600 nm bis 700 nm. Der Vorteil dieser Ausführungsform besteht darin, dass nur die gewünschte Strahlung reflektiert wird, es liegt also keine sonstige Streustrahlung vor. Allerdings handelt es sich um ein vergleichsweise kaltes Licht.

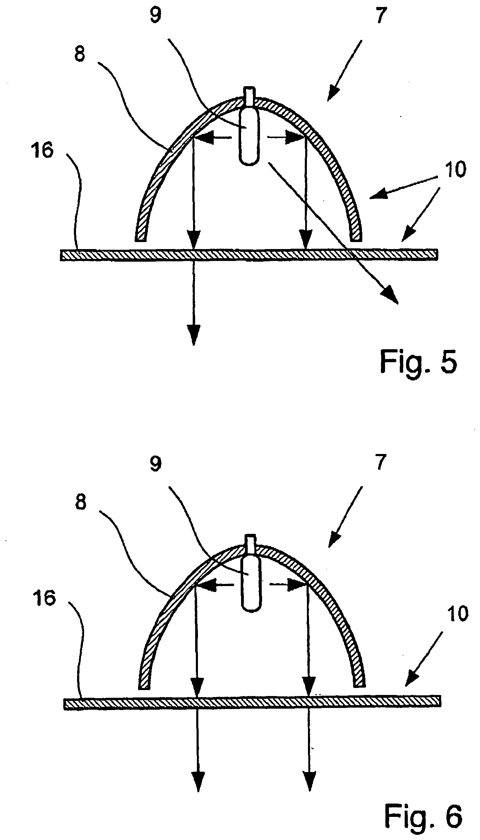

Bei der in Fig. 5 dargestellten Ausführungsform ist ein als Glasreflektor ausgebildeter Reflektor 8 vorgesehen. Der Glasreflektor reflektiert Strahlung im Wellenlängenbereich zwischen 580 und 710 nm. Auch hier gilt, dass innerhalb der angegebenen Bereichsgrenzen alle Teilbereiche möglich sind. Darüber hinaus ist eine Filterscheibe 16 vorgesehen, die Strahlung im Bereich größer 580 nm transmittiert. Hierbei ist es so, dass über den Glasreflektor reflektierte Strahlung, die durch die Filterscheibe 16 gelangt, im gewünschten Wellenlängenbereich abgegeben wird. Strahlung (Streulicht), die nicht über den Glasreflektor reflektiert wird und durch die Filterscheibe 16 gelangt, weist eine Wellenlänge von größer 580 nm auf. Diese Ausführungsform zeichnet sich dadurch aus, dass sie in einfacher Weise in eine bestehende Sonnenbank eingebaut werden kann. Aufgrund des Streulichtanteils ist das Licht nicht so kalt wie bei der Ausführungsform gemäß Fig. 4.

Bei der Ausführungsform gemäß Fig. 6 ist ein als Alureflektor ausgebildeter Reflektor 8 vorgesehen, der im Wellenlängenbereich größer 200 nm reflektiert. Des Weiteren ist eine Filterscheibe 16 vorgesehen, die im Wellenlängenbereich größer 580 nm transmittiert. Damit ergibt sich hinter der Filterscheibe 16 eine Strahlung im Wellenlängenbereich größer 580 nm.

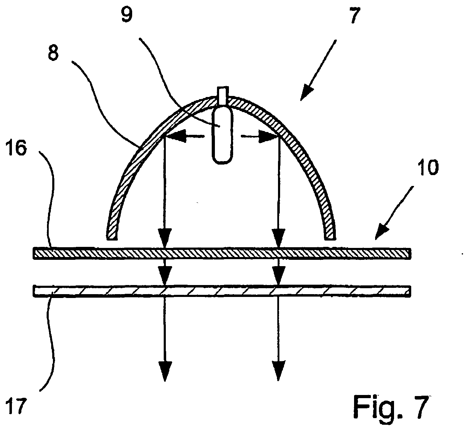

Die in Fig. 7 dargestellte Ausführungsform entspricht der in Fig. 6 dargestellten, wobei eine weitere Filterscheibe 17 vorgesehen ist. Diese blockt Strahlung im Bereich größer 710 nm, vorzugsweise größer 700 nm. Auf diese Weise ergibt sich eine transmittierte Strahlung durch beide Filterscheiben 16, 17 im Wellenlängenbereich zwischen 580 nm und 710 nm bis 700 nm.

Es versteht sich, dass die dargestellten Reflektoreinrichtungen 7 nur bevorzugte Ausführungsbeispiele darstellen und dass weitere Reflektoreinrichtungen 7 mit entsprechender Filtereinrichtung 10 ohne weiteres möglich sind.

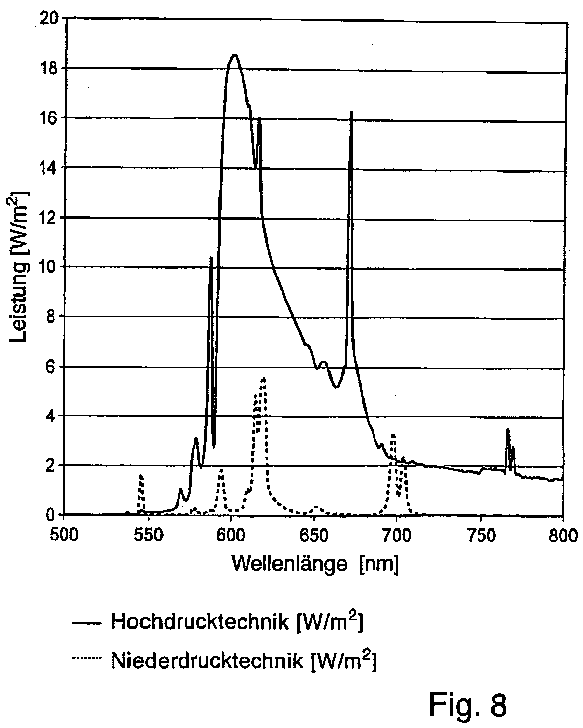

In Fig. 8 ist ein Diagramm dargestellt, bei dem die Strahlungsleistung in W/m² auf der Y-Achse gegen die Wellenlänge λ in nm auf der X-Achse aufgetragen sind. Dabei bezieht sich die Strahlungsleistung auf einen Abstand von wenigstens 30 cm oder mehr von der Bestrahlungslampe 9. Letztlich soll dies diejenige Strahlungsleistung wiedergeben, die auf einen Nutzer wirkt, der auf einer Liegefläche 3 einer Bestrahlungsvorrichtung 1 liegt und von einer Reflelctoreinrichtung 7 bzw. der zugehörigen Bestrahlungslampe 9 bestrahlt wird.

Dargestellt ist in Fig. 8 in durchgezogener Linie zum einen die Strahlungsleistung einer erfindungsgemäßen Bestrahlungsvorrichtung 1, während gestrichelt die Bestrahlungsleistung einer zum Stand der Technik gehörenden Lampe, die mit der Niederdrucktechnik arbeitet, dargestellt ist.

Nachfolgend wird zunächst auf die Strahlungsleistung der erfindungsgemäßen Bestrahlungsvorrichtung 1 eingegangen. Erkennbar weist die gefilterte Strahlung kaum Anteile im Bereich kleiner 550 nm auf. Der erste deutliche und steile Anstieg der Strahlungsleistung liegt kurz vor 600 nm, bevorzugt bei etwa 585 nm. Das Maximum mit über 18 W/m² befindet sich im Bereich von etwa 600 nm. Bis auf eine Spitze im Bereich von etwa 680 nm, die aufgrund der Zusammensetzung des Füllgases der Bestrahlungslampe 9 nicht vermeidbar, jedoch an sich ungewollt ist, ergibt sich ein im Wesentlichen konstanter Abfall der Strahlungsleistung bis etwa 700 nm. Im Bereich von etwa 700 nm liegt die Strahlungsleistung noch immer bei etwa 2 W/m² und nimmt dann aber im Wesentlichen stetig mit kleiner Steigung ab. Letztlich liegt die Strahlungsleistung der gefilterten Strahlung der Bestrahlungslampe 9 gemessen im Abstand von 30 cm von der Schutzscheibe 12 im Wellenlängenbereich zweischen 590 bis 700 nm bei etwa 890 W/m². Dem gegenüber liegt die Strahlungsleistung der zum Stand der Technik gehörenden Niederdrucklampe im Wellenlängenbereich zwischen 590 nm und 700 nm bei unter 80 W/m².

Die Fig. 8 verdeutlicht im Übrigen, dass die Strahlungsleistung einer zum Stand der Technik gehörenden Niederdrucklampe (gestrichelt dargestellt) nicht nur um ein Vielfaches geringer ist als bei der erfindungsgemäßen Bestrahlungslampe 9, es sind auch nicht unerhebliche Strahlungsanteile unterhalb von 550 nm und auch, was nicht dargestellt ist, im UV-Bereich vorgesehen, was bei der erfindungsgemäßen Bestrahlungslampe 9 nicht der Fall ist. Jedenfalls lassen sich mit der Bestrahlungsleistung der bekannten Niederdruck-Lampe keine oder nur geringe Hautverbesserungseffekte der eingangs genannten Art erzielen, während mit der erfindungsgemäßen Bestrahlungslampe 9 ganz erhebliche und unerwartet gute Hautverbesserungseffekte verbunden sind.

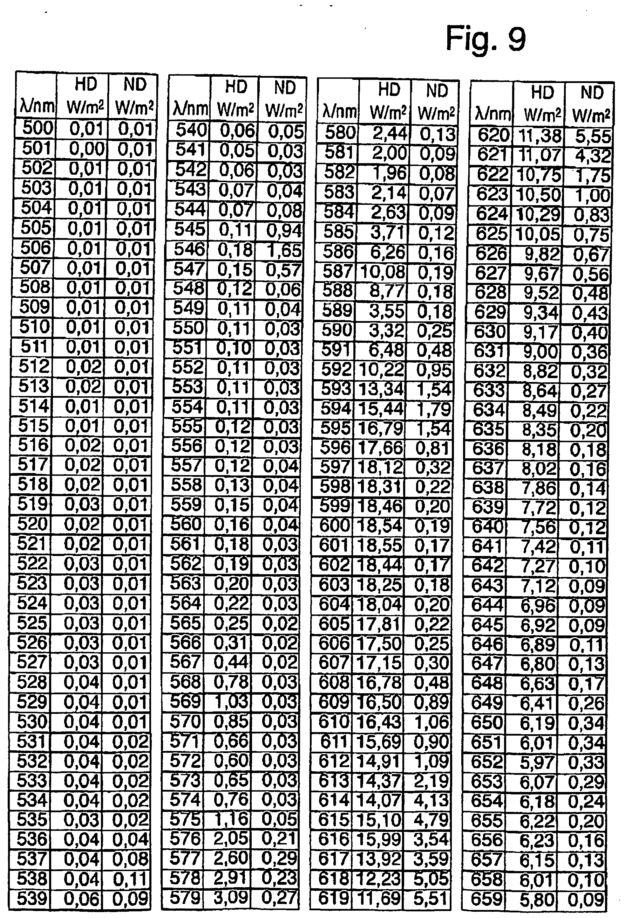

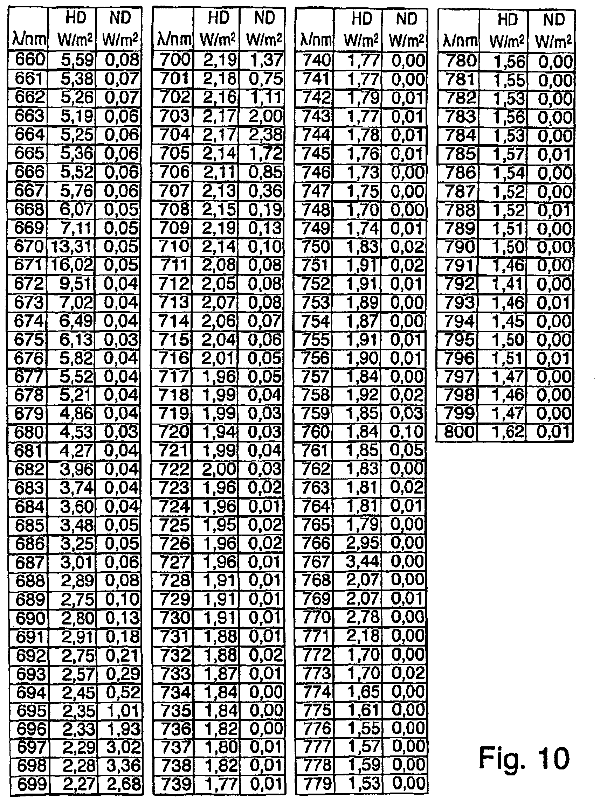

In den Fig. 9 und 10 sind jeweils in Form einer Tabelle die einzelnen Strahlungsleistungen im Wellenlängenbereich zwischen 500 nm und 800 nm angegeben, die zu den in Fig. 8 angegebenen Kurven führen. Dabei ist die Wellenlänge λ in nm und die jeweilige Strahlungsleistung in W/m² bei der erfindungsgemäßen Bestrahlungsvorrichtung 1 (HD für Hochdruck) und der bekannten Niederdrucklampe (ND für Niederdruck) angegeben. Aus den Tabellen ergibt sich, dass das Maximum der Strahlungsleistung bei der erfindungsgemäßen Bestrahlungsvorrichtung 1 bei 601 nm liegt. Werte über 18 W/m² ergeben sich im Wellenlängenbereich zwischen 597 nm bis 604 nm.

Bei der bekannten Niederdrucklampe liegt das Maximum mit 4,79 W/m² bei 615 nm. Erkennbar ergibt sich bei der erfindungsgemäßen Bestrahlungsvorrichtung 1 nicht nur ein etwa um den Faktor 4 höheres Strahlungsmaximum, auch das Integral der Strahlungsleistung im Bereich zwischen 590 nm und 700 nm differiert um mehr als den Faktor 10.

### Bezugszeichenliste:

- 1: Bestrahlungsvorrichtung
- 2: Unterbau
- 3: Liegefläche
- 4: Oberteil
- 5: Lampe
- 6: Gesichtsbereich
- 7: Reflektoreinrichtung
- 8: Reflektor
- 9: Bestrahlungslampe
- 10: Filtereinrichtung
- 11: Schutzscheibe
- 12: Luftspalt
- 13: Kühlung
- 14: Kühlung
- 15: Filterspiegel
- 16: Filterscheibe
- 17: Filterscheibe

## Patentansprüche

1. Bestrahlungsvorrichtung (1) mit wenigstens einer Bestrahlungslampe (9) zur Bestrahlung der menschlichen Haut mit Strahlung zumindest im Rotlichtbereich, wobei die Bestrahlungslampe (9) als Hochdruck- oder Höchstdruck-Gasentladungslampe ausgebildet ist, wobei eine Filtereinrichtung (10) zur Filterung der von der Bestrahlungslampe (9) abgestrahlten Strahlung vorgesehen ist und wobei die Filtereinrichtung (10) Strahlung der Bestrahlungslampe (9) im Wellenlängenbereich kleiner 550 nm filtert,
**dadurch gekennzeichnet,**
**dass** die Strahlungsleistung der gefilterten Strahlung einer Bestrahlungslampe (9) gemessen im Abstand von mehr als 30 cm von der Bestrahlungslampe (9) größer 450 W/m² ist, wobei wenigstens eine einen Reflektor (8) aufweisende Reflektoreinrichtung (7) und eine der Reflektoreinrichtung (7) zugeordnete Kühleinrichtung vorgesehen sind, wobei die Bestrahlungslampe (9) im Bereich des Reflektors (8) angeordnet ist.

2. Bestrahlungsvorrichtung nach Anspruch 1, **dadurch gekennzeichnet, dass** nach Filterung mehr als 50%, vorzugsweise mehr als 70% und insbesondere mehr als 80% der Strahlungsleistung der gefilterten Strahlung im Wellenlängenbereich größer 550 nm anfällt.

3. Bestrahlungsvorrichtung (1) mit wenigstens einer Bestrahlungslampe (9) zur Bestrahlung der menschlichen Haut mit Strahlung zumindest im Rotlichtbereich, wobei die Bestrahlungslampe (9) als Hochdruck- oder Höchstdruck-Gasentladungslampe ausgebildet ist, wobei eine Filtereinrichtung (10) zur Filterung der von der Bestrahlungslampe (9) abgestrahlten Strahlung vorgesehen ist und wobei die Filtereinrichtung (10) Strahlung der Bestrahlungslampe (9) im Wellenlängenbereich kleiner 550 nm filtert, **dadurch gekennzeichnet, dass** die Strahlungsleistung der gefilterten Strahlung einer Bestrahlungslampe (9) gemessen im Abstand von mehr als 30 cm von der Bestrahlungslampe (9) größer 450 W/m² ist, wobei nach Filterung mehr als 50%, vorzugsweise mehr als 70% und insbesondere mehr als 80% der Strahlungsleistung der gefilterten Strahlung im Wellenlängenbereich größer 550 nm anfällt.

4. Bestrahlungsvorrichtung nach einem der vorhergehenden Ansprüche, **dadurch gekennzeichnet, dass** weniger als 20%, vorzugsweise weniger als 15% und insbesondere weniger als 10% der gefilterten Strahlung im Wellenlängenbereich kleiner 550 nm anfällt.

5. Bestrahlungsvorrichtung nach einem der vorhergehenden Ansprüche, **dadurch gekennzeichnet, dass** die Filtereinrichtung (10) Strahlung der Bestrahlungslampe (9) im Wellenlängenbereich kleiner 570 nm und/oder größer 750 nm, vorzugsweise größer 710 nm und insbesondere größer 700 nm filtert.

6. Bestrahlungsvorrichtung nach einem der vorhergehenden Ansprüche, **dadurch gekennzeichnet, dass** die Strahlungsleistung der gefilterten Strahlung einer Bestrahlungslampe (9) gemessen im Abstand von mehr als 30 cm von der Bestrahlungslampe (9) durchgängig
a) mehr als 2 W/m² im Wellenlängenbereich von 600 nm bis 670 nm beträgt und/oder
b) mehr als 4 W/m² im Wellenlängenbereich von 600 nm bis 660 nm beträgt und/oder
c) mehr als 6 W/m² im Wellenlängenbereich von 600 nm bis 630 nm beträgt und/oder
d) mehr als 8 W/m² im Wellenlängenbereich von 600 nm bis 620 nm beträgt und/oder
e) mehr als 10 W/m² im Wellenlängenbereich von 600 nm bis 615 nm beträgt und/oder
f) mehr als 12 W/m² im Wellenlängenbereich von 600 nm bis 610 nm beträgt und/oder
g) mehr als 14 W/m² im Wellenlängenbereich von 600 nm bis 605 nm beträgt und/oder
h) mehr als 16 W/m² im Wellenlängenbereich von 600 nm bis 604 nm beträgt und/oder
i) mehr als 17 W/m² im Wellenlängenbereich von 600 nm bis 603 nm beträgt.

7. Bestrahlungsvorrichtung nach einem der vorhergehenden Ansprüche, **dadurch gekennzeichnet, dass** die Strahlungsleistung der gefilterten Strahlung einer Bestrahlungslampe (9) gemessen im Abstand von mehr als 30 cm von der Bestrahlungslampe (9) im Wellenlängenbereich zwischen 590 nm und 700 nm größer 500 W/m², vorzugsweise größer 700 W/m² und insbesondere größer 800 W/m² ist.

8. Bestrahlungsvorrichtung nach einem der vorhergehenden Ansprüche, **dadurch gekennzeichnet, dass** die Filtereinrichtung (10) einen schräggestellten Filterspiegel (15) aufweist, der Strahlung im Wellenlängenbereich bis 550 nm, vorzugsweise bis 570 nm und insbesondere bis 580 nm und/oder im Wellenlängenbereich oberhalb 710 nm, insbesondere oberhalb 700 nm transmittiert und ansonsten reflektiert.

9. Bestrahlungsvorrichtung nach einem der vorhergehenden Ansprüche, **dadurch gekennzeichnet, dass** die Filtereinrichtung (10) eine Strahlung im Wellenlängenbereich von 550 nm, vorzugsweise bis 570 nm und insbesondere bis 580 nm filternde Filterscheibe (16, 17) aufweist.

10. Bestrahlungsvorrichtung nach einem der vorhergehenden Ansprüche, **dadurch gekennzeichnet, dass** die Filtereinrichtung (10) eine Strahlung im Wellenlängenbereich oberhalb 710 nm, insbesondere oberhalb 700 nm filternde weitere Filterscheibe (12) aufweist.

11. Bestrahlungsvorrichtung nach einem der vorhergehenden Ansprüche, **dadurch gekennzeichnet, dass** der Reflektor (8) eine Strahlung im Wellenlängenbereich von 550 nm, vorzugsweise bis 570 nm und insbesondere bis 580 nm und/oder im Wellenlängenbereich oberhalb von 700 nm transmittiert und ansonsten reflektiert.

12. Bestrahlungsvorrichtung nach einem der vorhergehenden Ansprüche, **dadurch gekennzeichnet, dass** die Bestrahlungsvorrichtung (1) als Tunnelgerät mit Unterbau (2) und am Unterbau (2) angelenktem, verschwenkbaren Oberbau (4) ausgebildet ist und dass wenigstens eine Reflektoreinrichtung (7) im Oberbau (4) im Gesichtsbereich (6) vorgesehen ist.

13. Bestrahlungsvorrichtung nach einem der vorhergehenden Ansprüche, **dadurch gekennzeichnet, dass** die Reflektoreinrichtung (7) über einen Luftspalt (12) von einer strahlungsdurchlässigen Schutzscheibe (11) getrennt ist und dass eine weitere Kühleinrichtung zur Durchführung eines Kühlmediums durch den Luftspalt (12) vorgesehen ist.

14. Bestrahlungsvorrichtung nach einem der vorhergehenden Ansprüche, **dadurch gekennzeichnet, dass** ein regelbares oder nicht regelbares, insbesondere magnetisches oder elektronisches Vorschaltgerät vorgesehen ist und dass, vorzugsweise, die Bestrahlungslampe (9) mittels des Vorschaltgeräts dimmbar ist.

15. Bestrahlungsvorrichtung nach einem der vorhergehenden Ansprüche, **dadurch gekennzeichnet, dass** das Füllgas der Bestrahlungslampe (9) Quecksilber und/oder Xenon und/oder Natriumjodid und/oder Scandiumjodid aufweist.

## Claims

1. A radiation device (1) having at least one radiation lamp (9) for radiating human skin, having a radiation at least within the red light range, wherein the radiation lamp (9) is embodied as a high-pressure or highest-pressure gas discharge lamp, wherein a filter unit (10) is provided for filtering the radiation emitted by the radiation lamp (9), and wherein the filter unit (10) filters radiation from the radiation lamp (9) within a wavelength range of less than 550 nm,
**characterized in that**
the radiation output of the filtered radiation of a radiation lamp (9), measured at a distance of greater than 30 cm from the radiation lamp (9), is greater than 450 W/m², wherein at least one reflector unit (7) having a reflector (8), and a cooling unit associated with the reflector unit (7) are provided, wherein the radiation lamp (9) is disposed within the region of the reflector (8).

2. The radiation device according to claim 1, **characterized in that** after filtration more than 50%, preferably more than 70%, and in particular more than 80% of the radiation output of the filtered radiation is accrued within the wavelength range of greater than 550 nm.

3. A radiation device (1) having at least one radiation lamp (9) for radiating human skin, having a radiation at least within the red light range, wherein the radiation lamp (9) is embodied as a high-pressure or highest-pressure gas discharge lamp, wherein a filter unit (10) is provided for filtering the radiation emitted by the radiation lamp (9), and wherein the filter unit (10) filters radiation from the radiation lamp (9) within a wavelength range of less than 550 nm,
**characterized in that**
the radiation output of the filtered radiation of a radiation lamp (9), measured at a distance of greater than 30 cm from the radiation lamp (9), is greater than 450 W/m², wherein after filtration more than 50%, preferably more than 70%, and in particular more than 80% of the radiation output of the filtered radiation is accrued within the wavelength range of greater than 550 nm.

4. The radiation device according to one of the preceding claims, **characterized in that** less than 20%, preferably less than 15%, and in particular less than 10% of the filtered radiation is accrued within the wavelength range of less than 550 nm.

5. The radiation device according to one of the preceding claims, **characterized in that** the filter unit (10) filters the radiation of the radiation lamp (9) within the wavelength range of less than 570 nm and/or greater than 750 nm, preferably greater than 710 nm, and in particular greater than 700 nm.

6. The radiation device according to one of the preceding claims, **characterized in that** the radiation output of the filtered radiation of a radiation lamp (9), measured at a distance of greater than 30 cm from the radiation lamp (9), is consistently
a) greater than 2 W/m² within the wavelength range of 600 nm to 670 nm, and/or
b) greater than 4 W/m² within the wavelength range of 600 nm to 660 nm, and/or
c) greater than 6 W/m² within the wavelength range of 600 nm to 630 nm, and/or
d) greater than 8 W/m² within the wavelength range of 600 nm to 620 nm, and/or
e) greater than 10 W/m² within the wavelength range of 600 nm to 615 nm, and/or
f) greater than 12 W/m² within the wavelength range of 600 nm to 610 nm, and/or
g) greater than 14 W/m² within the wavelength range of 600 nm to 605 nm, and/or
h) greater than 16 W/m² within the wavelength range of 600 nm to 604 nm, and/or
i) greater than 17 W/m² within the wavelength range of 600 nm to 603 nm.

7. The radiation device according to one of the preceding claims, **characterized in that** the radiation output of the filtered radiation of a radiation lamp (9), measured at a distance of greater than 30 cm from the radiation lamp (9), is within the wavelength range of between 590 nm and 700 nm, greater than 500 W/m², preferably greater than 700 W/m², and in particular greater than 800 W/m².

8. The radiation device according to one of the preceding claims, **characterized in that** the filter unit (10) comprises a tilted filter mirror (15) which transmits the radiation within the wavelength range of up to 550 nm, preferably up to 570 nm, and in particular up to 580 nm, and/or within the wavelength range of above 710 nm, in particular above 700 nm, and otherwise reflects the same.

9. The radiation device according to one of the preceding claims, **characterized in that** the filter unit (10) comprises a filter disk (16, 17) filtering radiation within the wavelength range of 550 nm, preferably up to 570 nm, and in particular up to 580 nm.

10. The radiation device according to one of the preceding claims, **characterized in that** the filter unit (10) comprises a filter disk (12) filtering radiation within the wavelength range of above 710 nm, in particular above 700 nm.

11. The radiation device according to one of the preceding claims, **characterized in that** the reflector (8) transmits radiation within the wavelength range of 550 nm, preferably up to 570 nm, and in particular up to 580 nm and/or within the wavelength range of above 700 nm, and otherwise reflects the same.

12. The radiation device according to one of the preceding claims, **characterized in that** the radiation device (1) is embodied as a tunnel device having a substructure (2) and a pivoting superstructure (4) articulated on the substructure (2), and that at least one reflector unit (7) is provided in the superstructure (4) in the region of the face (6).

13. The radiation device according to one of the preceding claims, **characterized in that** the reflector unit (7) is separated from a radiation permeable protective shield (11) via an air gap (12), and that an additional cooling unit is provided for guiding a cooling medium through the air gap (12).

14. The radiation device according to one of the preceding claims, **characterized in that** a controllable or non-controllable, in particular magnetic or electronic series connection unit is provided and that preferably the radiation lamp (9) is dimmable via the series connection device.

15. The radiation device according to one of the preceding claims, **characterized in that** the filling gas of the radiation lamp (9) comprises mercury and/or xenon and/or sodium iodide and/or scandium iodide.

## Revendications

1. Équipement d'irradiation (1), comportant au moins une lampe d'irradiation (9) destinée à irradier la peau humaine avec un rayonnement au moins dans le domaine de la lumière rouge, la lampe d'irradiation (9) étant configurée comme une lampe à décharge dans un gaz haute pression ou très haute pression, un dispositif de filtrage (10) étant prévu pour filtrer le rayonnement émis par la lampe d'irradiation (9), le dispositif de filtrage (10) filtrant le rayonnement de la lampe d'irradiation (9) dans la plage de longueurs d'onde inférieure à 550 nm,
**caractérisé en ce que**
la puissance rayonnante du rayonnement filtré d'une lampe d'irradiation (9), mesurée à une distance supérieure à 30 cm par rapport à la lampe d'irradiation (9), est supérieure à 450 W/m², au moins un dispositif réflecteur (7), comprenant un réflecteur (8), et un dispositif de refroidissement, affecté au dispositif réflecteur (7), étant prévus, la lampe d'irradiation (9) étant disposée dans la zone du réflecteur (8).

2. Équipement d'irradiation selon la revendication 1, **caractérisé en ce que**, après le filtrage, plus de 50 %, de préférence plus de 70 % et en particulier plus de 80 % de la puissance rayonnante du rayonnement filtré apparaissent dans la plage de longueurs d'onde inférieure à 550 nm.

3. Équipement d'irradiation (1), comportant au moins une lampe d'irradiation (9) destinée à irradier la peau humaine avec un rayonnement au moins dans le domaine de la lumière rouge, la lampe d'irradiation (9) étant configurée comme une lampe à décharge dans un gaz haute pression ou très haute pression, un dispositif de filtrage (10) étant prévu pour filtrer le rayonnement émis par la lampe d'irradiation (9), le dispositif de filtrage (10) filtrant le rayonnement de la lampe d'irradiation (9) dans la plage de longueurs d'onde inférieure à 550 nm,
**caractérisé en ce que**
la puissance rayonnante du rayonnement filtré d'une lampe d'irradiation (9), mesurée à une distance supérieure à 30 cm par rapport à la lampe d'irradiation (9), est supérieure à 450 W/m², et après le filtrage, plus de 50 %, de préférence plus de 70 % et en particulier plus de 80 % de la puissance rayonnante du rayonnement filtré apparaissant dans la plage de longueurs d'onde inférieure à 550 nm.

4. Équipement d'irradiation selon l'une des revendications précédentes, **caractérisé en ce que** moins de 20 %, de préférence moins de 15 % et en particulier moins de 10 % du rayonnement filtré apparaissent dans la plage de longueurs d'onde inférieure à 550 nm.

5. Équipement d'irradiation selon l'une des revendications précédentes, **caractérisé en ce que** le dispositif de filtrage (10) filtre le rayonnement de la lampe d'irradiation (9) dans la plage de longueurs d'onde inférieure à 570 nm et/ou supérieure à 750 nm, de préférence supérieure à 710 nm et en particulier supérieure à 700 nm.

6. Équipement d'irradiation selon l'une des revendications précédentes, **caractérisé en ce que** la puissance rayonnante du rayonnement filtré d'une lampe d'irradiation (9), mesurée à une distance de plus de 30 cm par rapport à la lampe d'irradiation (9), est en permanence:
a) supérieure à 2 W/m² dans la plage de longueurs d'onde de 600 à 670 nm, et/ou
b) supérieure à 4 W/m² dans la plage de longueurs d'onde de 600 à 660 nm, et/ou
c) supérieure à 6 W/m² dans la plage de longueurs d'onde de 600 à 630 nm, et/ou
d) supérieure à 8 W/m² dans la plage de longueurs d'onde de 600 à 620 nm, et/ou
e) supérieure à 10 W/m² dans la plage de longueurs d'onde de 600 à 615 nm, et/ou
f) supérieure à 12 W/m² dans la plage de longueurs d'onde de 600 à 610 nm, et/ou
g) supérieure à 14 W/m² dans la plage de longueurs d'onde de 600 à 605 nm, et/ou
h) supérieure à 16 W/m² dans la plage de longueurs d'onde de 600 à 604 nm, et/ou
i) supérieure à 17 W/m² dans la plage de longueurs d'onde de 600 à 603 nm.

7. Équipement d'irradiation selon l'une des revendications précédentes, **caractérisé en ce que** la puissance rayonnante du rayonnement filtré d'une lampe d'irradiation (9), mesurée à une distance supérieure à 30 cm par rapport à la lampe d'irradiation (9), est, dans la plage de longueurs d'onde comprise entre 590 et 700 nm, supérieure à 500 W/m², de préférence supérieure à 700 W/m², et en particulier supérieure à 800 W/m².

8. Équipement d'irradiation selon l'une des revendications précédentes, **caractérisé en ce que** le dispositif de filtrage (10) comprend un miroir filtrant (15) en position inclinée, qui transmet et sinon réfléchit le rayonnement dans la plage de longueurs d'onde allant jusqu'à 550 nm, de préférence jusqu'à 570 nm et en particulier jusqu'à 580 nm, et/ou dans la plage de longueurs d'onde supérieure à 710 nm, en particulier supérieure à 700 nm.

9. Équipement d'irradiation selon l'une des revendications précédentes, **caractérisé en ce que** le dispositif de filtrage (10) comprend une lame filtrante (16, 17), qui filtre le rayonnement dans la plage de longueurs d'onde de 550 nm, de préférence allant jusqu'à 570 nm et en particulier allant jusqu'à 580 nm.

10. Équipement d'irradiation selon l'une des revendications précédentes, **caractérisé en ce que** le dispositif de filtrage comprend une lame filtrante (12) supplémentaire, qui filtre le rayonnement dans la plage de longueurs d'onde supérieure à 710 nm, en particulier supérieure à 700 nm.

11. Équipement d'irradiation selon l'une des revendications précédentes, **caractérisé en ce que** le réflecteur (8) transmet et sinon réfléchit un rayonnement dans la plage de longueurs d'onde de 550 nm, en particulier allant jusqu'à 570 nm et en particulier jusqu'à 580 nm et/ou dans la plage de longueurs d'onde supérieures à 700 nm.

12. Équipement d'irradiation selon l'une des revendications précédentes, **caractérisé en ce que** l'équipement d'irradiation (1) est configuré comme un équipement tunnel, avec une partie inférieure (2) et une partie supérieure (4), articulée à la partie inférieure (2) et pouvant pivoter, et **en ce qu'**au moins un dispositif réflecteur (7) est prévu dans la partie supérieure (4), dans la zone du visage (6).

13. Équipement d'irradiation selon l'une des revendications précédentes, **caractérisé en ce que** le dispositif réflecteur (7) est, par l'intermédiaire d'un espace d'air (12), séparé d'une lame de protection (11), perméable au rayonnement, et **en ce qu'**un dispositif de refroidissement supplémentaire est prévu pour faire passer un fluide de refroidissement à travers l'espace d'air (12).

14. Équipement d'irradiation selon l'une des revendications précédentes, **caractérisé en ce qu'**est prévu un ballast réglable ou non réglable, en particulier magnétique ou électronique, et **en ce qu'**en particulier la lampe d'irradiation (9) peut être atténuée à l'aide du ballast.

15. Équipement d'irradiation selon l'une des revendications précédentes, **caractérisé en ce que** le gaz de remplissage de la lampe d'irradiation (9) comprend du mercure et/ou du xénon et/ou de l'iodure de sodium et/ou de l'iodure de scandium.
